# EUROPEAN PATENT SPECIFICATION

(11) **EP 2 004 208 B1**
(45) Date of publication and mention of the grant of the patent: **25.01.2017**
(21) Application number: 06848025.0
(22) Date of filing: 21.12.2006
(51) Int. Cl.: A61K 36/74

(54) **SKIN PROTECTION AND IMPROVEMENT**
HAUTSCHUTZ UND -VERBESSERUNG
PROTECTION ET AMELIORATION DE LA PEAU

(30) Priority: 23.12.2005 US 754006 P
(43) Date of publication of application: 24.12.2008
(73) Proprietor: MARS, INCORPORATED, McLean, Virginia 22101-3883 (US)
(72) Inventor: SIES, Helmut, D-40667 Meersbusch (DE); STAHL, Wilhelm, D-40545 Dusseldorf (DE)
(74) Representative: Keen, Celia Mary
(86) International application number: PCT/US2006/049005
(87) International publication number: WO 2007/076006

(56) References cited:
- WO-A-01/62265
- WO-A-99/44578
- WO-A-99/45797
- US-A1- 2005 267 052
- US-B2- 6 517 841
- SALIOU CLAUDE ET AL: "Solar ultraviolet-induced erythema in human skin and nuclear factor-kappa-B-dependent gene expression in keratinocytes are modulated by a French maritime pine bark extract" FREE RADICAL BIOLOGY AND MEDICINE, vol. 30, no. 2, 15 January 2001 (2001-01-15), pages 154-160, XP002535456 ISSN: 0891-5849
- SIES HELMUT ET AL: "Nutritional protection against skin damage from sunlight" ANNUAL REVIEW OF NUTRITION, vol. 24, 2004, pages 173-200, XP002535457 ISSN: 0199-9885
- SPENCER ET AL.: 'Epicatechin and its in vivo metabolite, 3'-O-methyl epicatechin, protect human fibroblasts from oxidative-stress-induced cell death involving caspase-3 activation' BIOCHEM. J. vol. 354, no. 3, 15 March 2001, pages 493 - 500, XP007908745
- NEUKAM ET AL.: 'Consumption of flavanol-rich cocoa acutely increases microcirculation in human skin' EUR. J. NUTR. vol. 46, no. 1, 11 December 2006, pages 43 - 56, XP019491014
- HEINRICH ET AL.: 'Long-Term Ingestion of High Flavanol Cocoa Provides Photoprotection against UV-induced Erythema and Improves Skin Condition in Women' J. NUTR. vol. 136, no. 6, June 2006, pages 1565 - 1569, XP002545382

## Description

### FIELD OF THE INVENTION

This invention relates to methods for improving skin quality, comprising orally administering to a subject in need thereof certain polyphenolic compounds described herein.

### BACKGROUND OF THE INVENTION

Skin is the largest organ of the body, serving as a protective shield against light (e.g. UV radiation), heat, mechanical damage/injury, noxious substances and microbial infections. Skin also plays an important functional role in regulating body homeostasis by regulating body temperature, water stores, lipids and vitamin D. Furthermore, the skin plays a pivotal role in the feeling of well-being and physical attractiveness.

Several exogenous/extrinsic (e.g. environmental conditions) and endogenous/intrinsic (e.g. genetic predisposition, immune status, hormonal status and stress) factors affect skin properties such as structure, texture, thickness, density, hydration, color, and its shielding properties. Exposure to environmental factors such as extremes of temperature, wind and sunlight/solar radiation can compromise skin properties, appearance, and functioning, leading to the deterioration of skin quality, reduction in skin attractiveness and accelerated skin aging. Therefore, there is a need in the art for methods for improving skin quality.

The nutritional status of the organism affects skin quality and functioning and optimal supply with macro- and micro-nutrients may contribute to skin health. Applicants have now discovered that oral consumption of the compounds recited herein has photoprotective effects (e.g. reducing skin erythema and photoaging) on skin exposed to UV radiation as well as beneficial effects on skin quality (e.g. structure, texture, hydration).

### SUMMARY OF THE INVENTION

The invention relates to non-therapeutic methods for improving skin quality, comprising orally administering to a subject in need thereof certain polyphenolic compounds described herein.

A polyphenolic compound described herein may be included in a composition such as a food (including pet food), a food additive, a dietary supplement, or a pharmaceutical. Packaged products containing the above- mentioned compositions and a label and/or instructions for use to improve skin quality are also used in the invention.

In one aspect the invention relates to a non-therapeutic method of improving skin quality, for example skin structure, texture and hydration, by orally administering to a subject in need thereof an effective amount of a polyphenolic compound described herein.

### DETAILED DESCRIPTION

The invention relates to a non-therapeutic method for improving skin quality, comprising orally administering to a subject in need thereof certain polyphenolic compounds described herein. Accordingly, the present invention provides a non-therapeutic method of improving skin quality in a subject by reducing skin roughness, increasing skin thickness or increasing skin density, the method comprising orally administering to the subject a composition which comprises an effective amount of a cocoa component wherein the cocoa component comprises:
(i) a compound selected from epicatechin and catechin; or
(ii) a compound having the formula A, or a pharmaceutically acceptable salt thereof: 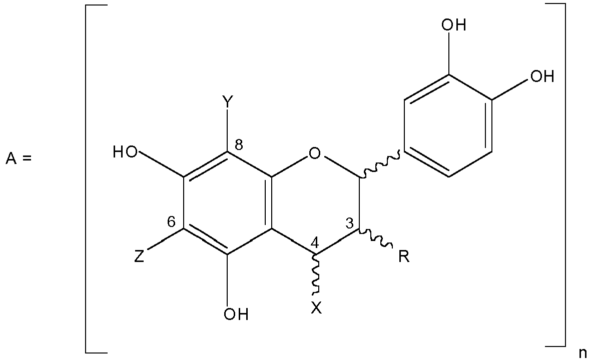 wherein
   n is an integer from 2 to 18;
   R and X each have either α or β stereochemistry;
   R is OH or O-sugar;
   the substituents of C-4, C-6 and C-8 are X, Z and Y, respectively, and bonding of monomeric units occurs at C-4, C-6 or C-8;
   when any C-4, C-6 or C-8 is not bonded to another monomeric unit, X, Y and Z independently are hydrogen or a sugar; and
   the sugar is optionally substituted with a phenolic moiety at any position, for instance, via an ester bond.

The compounds for use in the present invention include certain flavanols (flavan-3-ols), procyanidins, or pharmaceutically acceptable salts thereof. Such compounds, when of natural origin, may be included in the composition in the form of a cocoa component, for example cocoa nibs or fragments thereof, chocolate liquor, partially and fully-defatted cocoa solids, cocoa extract or fraction thereof.

As used herein, the term "flavanol" or "flavan-3-ol" refers to a monomer of the following formula:

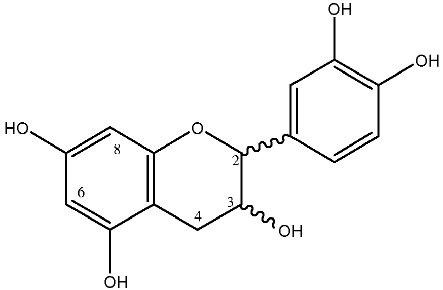

The term "procyanidin" refers to an oligomer.

The term "cocoa component" refers to a component derived from cocoa bean, e.g. cocoa nibs and fragments thereof, chocolate liquor, partially and fully-defatted cocoa solids (e. g. cake or powder), flavanol and/on procyanidin-containing cocoa extract or fraction thereof.

In certain embodiments of the present invention a flavanol (e.g. (-)-epicatechin and (+)-catechin) may be used, or a composition comprising an effective amount of the flavanol (e.g. (-)-epicatechin and (+)-catechin), or a pharmaceutically acceptable salt thereof.

Other embodiments of the present invention use a compound, or a composition comprising an effective amount of the compound, having the following formula A, or a pharmaceutically acceptable salt thereof: 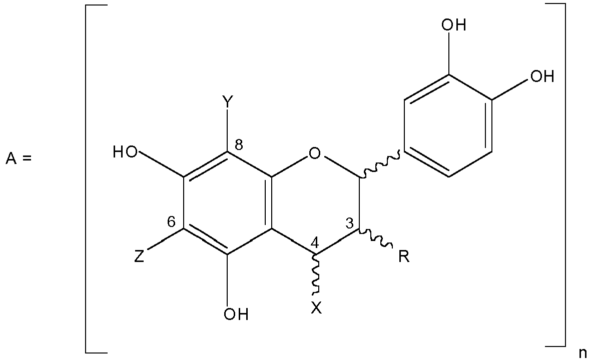 wherein
n is an integer from 2 to 18;
R and X each have either α or β stereochemistry;
R is OH or O-sugar;
the substituents of C-4, C-6 and C-8 are X, Z and Y, respectively, and bonding of monomeric units occurs at C-4, C-6 or C-8;
when any C-4, C-6 or C-8 is not bonded to another monomeric unit, X, Y and Z independently are hydrogen or a sugar; and
the sugar is optionally substituted with a phenolic moiety at any position, for instance, via an ester bond.

Monomeric units in the formula A may be bonded via 4→6α; 4→6β; 4→8α; and/or 4→8β linkages. The sugar is preferably a monosaccharide or a di- saccharide. The sugar may be selected from the group consisting of glucose, galactose, rhamnose, xylose, and arabinose. The phenolic moiety may be selected from the group consisting of caffeic, cinnamic, coumaric, ferulic, gallic, hydroxybenzoic and sinapic acids.

Examples of the compounds useful for method of the invention include the compounds of the formula A described herein wherein the integer n is 3 to 18; 2 to 12; 3 to 12; 2 to 5; 4 to 12; 5 to 12; 4 to 10; or 5 to 10. In some embodiments, the integer n is 2 to 4, for example 2 or 3. This disclosure applies to any compound of formula A herein.

### Methods of Use

The invention relates to non-therapeutic methods of improving skin quality, in a subject in need thereof.

As used herein, "improving skin quality" means achieving a measurable improvement of skin quality. The term "skin quality" refers to skin properties such as skin hydration (e.g. improvement of dryness), or skin texture (e.g. skin thickness, roughness, scaliness). For example, with respect to skin texture, the invention includes methods of reducing skin roughness, improving scaliness, and/or improving skin thickness in a subject in need thereof. A person of skill in the art will select the known methods of measuring the improvement of skin quality (e.g. methods described in the Examples).

In certain embodiments , the present invention provides a non-therapeutic method of improving skin quality, comprising orally administering to a mammal (e.g. human) or a veterinary animal in need thereof an effective amount of a flavanol such as epicatechin or catechin (e.g. (-)-epicatechin or (+)-catechin), or a pharmaceutically acceptable salt thereof.

The term "veterinary animal" refers to any animal cared for, or attended to by, a veterinarian, and includes companion (pet) animals and livestock animals, for example a cat, a dog and a horse.

For example, the above method may involve use of a compound A, or a pharmaceutically acceptable salt thereof, wherein R is OH, and when any C-4, C-6 or C-8 is not bonded to another monomeric unit, X, Y and Z are hydrogen. Examples of suitable sugars are as described above. Examples of phenolic moieties are as described above.

Examples of subjects in need of improvement of skin quality will be apparent to those of skill in the art, for example, the subjects that experience deterioration of skin properties which for example may lead to dryness, loss of thickness, density, and overall appearance. Such subjects may experience changes in skin quality due to exogenous/extrinsic (e.g. environmental conditions) and/or endogenous/intrinsic (e.g. genetic predisposition, immune status, hormonal status and stress) factors. Subjects exposed to, or about to be exposed to, environmental factors such as extremes of temperature, wind, or indoor conditions such as heating or air- conditioning benefit form the present invention.

The present compounds may be administered orally in the form of a cocoa component, for example cocoa nibs or fragments thereof, chocolate liquor, partially and fully-defatted cocoa solids (e.g. cocoa powder), cocoa extract or fraction thereof, or may be added independently of cocoa components. The cocoa component may be prepared such that the content of cocoa polyphenols (CP) is preserved.

In some embodiments, the present compounds may be administered in combination with other skin-protective agents and/or to enhance responsiveness to other skin-protective agents. Examples of skin-protective agents include vitamins, amino acids, minerals, micronutrients, botanical extracts or their derivatives, and herbs. These additional skin-protective agents may be administered either topically or orally.

Thus, the following uses are within the scope of the invention. Use of a flavanol, or a pharmaceutically acceptable salt thereof, as defined above, in the manufacture of a medicament, food, nutraceutical or dietary supplement for improving skin quality. Use of a compound of formula A, or a pharmaceutically acceptable salt thereof, as defined herein, in the manufacture of a medicament, food, nutraceutical or dietary supplement for use in improving skin quality.

The effective amount may be determined by a person of skill in the art using the guidance provided herein and general knowledge in the art. For example, the effective amount may be such as to achieve a physiologically relevant concentration in the body of a mammal. Such a physiologically relevant concentration may be at least 20 nanomolar (nM), preferably at least about 100 nM, and more preferably at least about 500 nM. In one embodiment, at least about one micromole in the blood of the mammal, such as a human, is achieved. The compounds defined herein, may be administered at from about 35 mg/day, 40 mg/day or 50 mg/day (e.g. to about 1000 mg/day), or from about 75 mg/day (e.g. to about 1000 mg/day), or from about 100-150 mg/day (e.g. to about 900 mg/day), or from about 300 mg/day (e.g. to about 500 mg/day). However, amounts higher than exemplified above may be used since the upper end of the amount range is not a limiting factor. The amounts may be measured as described in Adamson, G.E. et al., J. Ag. Food Chem.; 1999; 47 (10) 4184-4188.

The administration may be continued as a regimen, i.e., for an effective period of time, e.g., daily, monthly, bimonthly, biannually, annually, or in some other regimen, as determined by the skilled medical practitioner for such time as is necessary. The administration may be continued for at least a period of time required for improvement of skin quality. The composition may be administered daily, preferably two or three times a day, for example, morning and evening to maintain the levels of the effective compounds in the body of the mammal. To obtain the most beneficial results, the composition may be administered for at least 7 days, or at least 14 days, or at least 30 days, or at least 45 days, or at least 60 days, or at least 90 days. These regimens may be repeated periodically as needed.

### Compositions and Formulations

The compounds of the invention may be administered as a food (including pet food), a food additive, or a dietary supplement, or a pharmaceutical.

As used herein, "food" is a material containing protein, carbohydrate and/or fat, which is used in the body of an organism to sustain growth, repair and vital processes and to furnish energy. Foods may also contain supplementary substances, for example, minerals, vitamins and condiments. See Merriam- Webster's Collegiate Dictionary, 10th Edition, 1993. The term food includes a beverage adapted for human or animal consumption. As used herein a "food additive" is as defined by the FDA in 21 C.F.R.. 170.3(e)(1) and includes direct and indirect additives. As used herein, a "dietary supplement" is a product (other than tobacco) that is intended to supplement the diet that bears or contains the one or more of the following dietary ingredients: a vitamin, a mineral, an herb or other botanical, an amino acid, a dietary substance for use by man to supplement the diet by increasing the total daily intake, or a concentrate, metabolite, constituent, extract or combination of these ingredients. As used herein, a "pharmaceutical" is a medicinal drug. See Merriam- Webster's Collegiate Dictionary, 10th Edition, 1993. A pharmaceutical may also be referred to as a medicament. The above compositions may be prepared as is known in the art.

The compositions may contain a carrier, a diluent, or an excipient. Depending on the intended use, the carrier, diluent, or excipient may be chosen to be suitable for human or veterinary use, food, additive, dietary supplement or pharmaceutical use. The composition may optionally contain an additional skin- protective agent. Also depending on use, a person of skill in the art may select the degree of purity of the compound of the invention. For example, when used to prepare pharmaceutical dosage forms, the compound should be as pure as commercially possible, while when preparing food, additive, or supplement, less pure or mixtures of compounds (e.g. plant extracts) may be used.

The compound of the invention may be "isolated and purified," i.e., it may be separated from compounds with which it naturally occurs (e.g. when the compound is of natural origin), or it may be synthetically prepared, in either case such that the level of contaminating compounds and/or impurities does not significantly contribute to, or detract from, the effectiveness of the compound. For example, an "isolated and purified B2 dimer" is separated from B5 dimer, with which it may occur in nature (e.g. in cocoa bean), to the extent achievable by the available commercially viable purification and separation techniques. Such compounds are particularly suitable for pharmaceutical applications.

The compound may also be less pure, i.e., "substantially pure," i.e., it may possess the highest degree of homogeneity achievable by available purification, separation and/or synthesis technology but need not be separated from the like compounds. As used herein, "the like compounds" are the compounds having the same degree of polymerization. For example, a "substantially pure dimer" refers to a mixture of dimers (e.g. B2 and B5, as it would occur in a cocoa extract fraction). While less suitable for pharmaceutical applications, such "substantially pure" compounds may be utilized for food, food additive and dietary supplement applications.

In some embodiments, the compound of the invention is at least 80% pure, at least 85% pure, at least 90% pure, at least 95% pure, at least 98% pure, or at least 99% pure. Such compounds are particularly suitable for pharmaceutical applications.

Pharmaceuticals containing the inventive compounds, optionally in combination with another skin-protective agent are administered orally. As used herein, "oral administration" includes administration by the mouth and includes sublingual and bucal administrations. A person of skill in the art will be able to determine a suitable mode of administration to maximize the delivery of the compounds of the invention. Thus, dosage forms adapted for each type of administration by mouth are within the scope of the invention and include solid, liquid and semi-solid dosage forms, such as tablets, capsules, gelatin capsules (gelcaps), bulk or unit dose powders or granules, emulsions, suspensions, pastes, or jellies. Sustained- release dosage forms are also within the scope of the invention. Suitable pharmaceutically acceptable carriers, diluents, or excipients are generally known in the art and can be determined readily by a person skilled in the art. The tablet, for example, may comprise an effective amount of the compound of the invention and optionally a carrier, such as sorbitol, lactose, cellulose, or dicalcium phosphate.

The foods comprising the compounds described herein and optionally another skin-protective agent may be adapted for human or veterinary use, and include pet foods. The food may be other than a confectionery, for example, a beverage (e.g. cocoa flavored beverage). A confectionery such as a standard of identity (SOI) and non-SOI chocolate, such as milk, sweet and semi-sweet chocolate including dark chocolate, low fat chocolate and a candy which may be a chocolate covered candy are also within the scope of the invention. Other examples include a baked product (e.g. brownie, baked snack, cookie, biscuit) a condiment, a granola bar, a toffee chew, a meal replacement bar, a spread, a syrup, a powder beverage mix, a cocoa or a chocolate flavored beverage, a pudding, a rice cake, a rice mix, a savory sauce and the like. If desired, the foods may be chocolate or cocoa flavored. Food products may be chocolates and candy bars, such as granola bars, containing nuts, for example, peanuts, walnuts, almonds, and hazelnuts. The food is designed to deliver an effective amount of the compounds described herein.

The compounds for use in the present invention may be of natural origin, for example, derived from a cocoa bean or another natural source known to a person of skill in the art, or prepared synthetically. A person of skill in the art may select natural or synthetic polyphenol based on the use and/or availability or cost.

The compounds may be included in the composition in the form of a cocoa component. For example, the compound(s) may be included in the composition in the form of a cocoa ingredient, for example, chocolate liquor included in chocolate, or may be added independently of cocoa ingredients, for example, as an extract, extract fraction, isolated and purified individual compound, pooled extract fractions or a synthetically prepared compound. The term "cocoa ingredient" refers to a cocoa solids-containing material derived from shell-free cocoa nibs such as chocolate liquor and partially or fully-defatted cocoa solids (e.g. cake or powder). The extraction and purification may be conducted as described in U.S. Pat. Nos. 5,554,645 and 6,670,390 to Romanczyk et ah, and U. S. Pat. No. 6,627,232 to Hammerstone et al..

Cocoa flavanols and/or procyanidins may be provided in the composition of the invention by cocoa ingredients containing these compounds or by including chocolate, which may be milk, sweet and semi-sweet, and is preferably dark chocolate, and low fat chocolate. The cocoa ingredients may be prepared using traditional cocoa processing procedures but is preferably prepared using the method described in U.S. Pat. No. 6,015,913 to Kealey et al. Alternatively, to enhance the level of cocoa polyphenols, chocolate liquor and cocoa solids prepared from cocoa beans having a fermentation factor of 275 or less may be used. These ingredients have cocoa polyphenol content that is higher than can be obtained using traditional cocoa processing methods (e.g. with roasting) and fully fermented beans. The chocolate may be prepared using conventional techniques from the ingredients described above or using an improved process for preserving cocoa polyphenols during chocolate manufacturing as described in WO99/45788 and in its U. S. counterpart, U. S. Pat. No. 6,194,020. A chocolate prepared by at least one of the following non-traditional processes is referred to herein as a "chocolate having a conserved amount of cocoa polyphenols": (i) preparing cocoa ingredients from underfermented or unfermented cocoa beans; (ii) preserving cocoa polyphenol during cocoa ingredient manufacturing process; and (iii) preserving cocoa polyphenol during chocolate manufacturing process. Such non-traditional processes may be used to prepare other cocoa component-containing products (foods e.g. beverages, dietary supplements) designed to contain enhanced levels of flavanols and/or procyanidins.

Synthetic procyanidins may also be used and are prepared by methods known in the art and as described, for example in, U. S. Pat. Nos. 6,420,572; 6,156,912; and 6,864,377.

A daily effective amount of the compound of the invention may be provided in a single serving in case of a food or a single dosage in case of a pharmaceutical or a dietary supplement. For example, a confectionery (e.g. chocolate) may contain at least about 100 mg/serving (e.g. 150-200, 200-400 mg/serving). (0048) The dietary supplement containing the compounds of the invention, and optionally another skin-protective agent, may be prepared using methods known in the art and may comprise, for example, nutrient such as dicalcium phosphate, magnesium stearate, calcium nitrate, vitamins, and minerals.

Further within the scope of the invention is an article of manufacture such as a packaged product comprising the composition of the invention (e.g. a food, a dietary supplement, a pharmaceutical) and a label indicating the presence of, or an enhanced content of the inventive compounds or directing use of the composition for improving skin quality. The packaged product may contain the composition and the instructions for use for improving skin quality. The label and/or instructions for use may refer to any of the methods of use described in this application.

The invention also relates to a method of manufacturing an article of manufacture comprising any of the compositions described herein, packaging the composition to obtain an article of manufacture and instructing, directing or promoting the use of the composition/article of manufacture for any of the uses described herein. Such instructing, directing or promoting includes advertising.

The invention is further described in the following non-limiting examples. Examples that fall outside the scope of the claims are included as reference embodiments.

### EXAMPLES

### Example 1: Effect of oral administration of high flavanol cocoa on skin

### Materials and Methods

Study design. A total of 24 volunteers between IS and 65 years old with healthy, normal skin of type II according to Fitzpatrick and Pathak (Pathak, M. A., J. Am. Acad. Dermatol, 1982, 7:285-312) were included in the study. Exclusion criteria were: pregnancy and breast-feeding, smoking, intake of medication that might influence the outcome of the study, sunbathing or the use of sun-beds, intake of vitamin supplements and of diets comprising a change of normal eating habits. Volunteers were randomly assigned to either the high flavanol group (HF) or the low flavanol group (LF) each consisting of 12 volunteers.

The HF group ingested a cocoa powder providing 326 mg of cocoa polyphenols per day over a period of 12 wk. The LF group ingested a matched cocoa powder providing 27 mg of polyphenols per day over the same period of time. The powder was dissolved in 100 ml of hot water and was ingested in the morning with a meal. Further details on the composition of the cocoa powder are given in Table 1.

**Table 1: Composition of the cocoa powder per serving dissolved in 100 mL water**

| Parameter (per unit) | high flavanol product (HF) | low flavanol product (LF) |
|---|---|---|
| | | |
| Calories | 53 kcal | 57 kcal |
| Fat (total) | 1.0 g | 1.0 g |
| Saturated fatty acids | - | - |
| Cholesterol | - | - |
| Sodium | 60 mg | 140 mg |
| Carbohydrates (total) | 9.0 g | 9.0 g |
| Fiber | 4.0 g | 4.0 g |
| Sugars Protein | 5.0 g | 5.0 g |
| | 5.0 g | 5.0 g |
| Caffeine | 10.6 mg | 12.3 mg |
| Theobromine | 195 mg | 190 mg |
| Cocoa polyphenols | 326 mg | 27 mg |
| (+)-Catechin | 12.7 mg | 1.2 mg |
| (-)-Epicatechin | 45.2 mg | 3.9 mg |

On day 0, wk 6, and wk 12 the following parameters related to photoprotection and skin health were determined: sensitivity towards UV-irradiation, cutaneous blood flow, skin structure and texture, skin hydration and transepidermal water loss.

Sensitivity towards UV-irradiation. The MED (minimal erythemal dose) was determined for each subject prior to the start of the study. Irradiation to induce erythema (1.25-fold the MED) was applied to dorsal skin (back, scapular region) using a blue-light solar simulator (Sol 3,Hönle, Munich, Germany). At each time point (wk 0, 6, 12) skin color was measured before and 24 h after irradiation. Skin color was evaluated by chromametry (Minolta CR 300, Ahrensburg, Germany) using the three-dimensional color system (L, a, b -values) (REF). L-values are a parameter for lightness of skin and b-values (blue/yellow axis) are indicative for pigmentation. a-Values (red/green-axis) are a measure for erythema and are used to quantify skin responses to UV irradiation. Decreasing a-values indicate a photoprotective effect.

Cutaneous blood flow, amount and oxygen saturation of hemoglobin. For measurement of peripheral blood flow and oxygen saturation of hemoglobin the O₂C-system (Lea Instruments, Giessen, Germany) was applied. The measurements of blood flow and velocity are based on the Doppler effect; the frequency of light is shifted by a moving erythrocyte depending on its velocity. Hemoglobin amount and oxygen saturation were determined spectroscopically. All parameters were measured in different skin layers (1 mm, 7-8 mm).

Skin structure and texture. High-frequency Ultrasound B-Scan (Frequency of 20 MHz -Derma Scan C, Vers. 3 Hersteller Ort etc) with 2-D-configuration (Cortex Technology, Denmark) was applied to analyze tissue structures and obtain information on skin density (Pixel) and thickness (mm) (Altmeyer P, el Gammal S, Hoffmann K (eds), Ultrasound in Dermatology, 1992, Springer Verlag, Berlin, Heidelberg). Skin surface profiles were evaluated with the SELS (Surface Evaluation of Living Skin) method (Visioscan, Courage & Khazaka Electronics, Cologne, Germany) in a 15x17 mm area. Four different parameters were applied to characterize the skin surface: roughness, scaling, smoothness, wrinkles.

Skin hydration and transepidermal water loss. Skin hydration (au) was determined by corneometry (Corneometer CM 825, Courage & Khazaka Electronics, Cologne, Germany); transepidermal waterloss (TEWL, g/hxm²) was measured using a TEWA-Meter TM 300 (Courage & Khazaka Electronics, Cologne, Germany) (Heinrich et al., Intern. J. Cosmet. Sci., 2003, 25:45-53;Rodrigues et al, Skin Res. Technol., 2004, 10:257-262). Statistics. For all parameters descriptive statistics (mean, standard deviation, minimum, lower quartile, median, upper quartile and maximum) were calculated for all time points (week 0, week 6, week 12). For all parameters pre-post differences for each combination of two time points were calculated. Within the two treatment groups each combination of two time points was compared using the Wilcoxon signed-rank test.

The pre-post differences of the two treatment groups were compared using the Wilcoxon rank-sum test.

### Results

The cocoa powders used in the high and low flavanol group were comparable with respect to their constituents except for the polyphenol content (see Table 1 above). In the HF group, a daily dose of 326 mg total polyphenols was ingested whereas only 27 mg of total polyphenols were provided with the cocoa powder in the LF group. The daily doses of (-)-epicatechin and (+)-catechin were 45.2 mg and 12.7 mg, respectively, in the HF group and 3.9 mg and 1.2 mg in the LF group.

Photoprotection. Protection of cocoa flavanols against UV-induced skin responses (erythema) was measured as a decrease in reddening following exposure of selected skin areas towards 1.25 MED. Reddening after UV exposure was determined by chromametry. Chromametry a-values 24 h after irradiation and the difference between chromametry a-values after and before irradiation (Δ-a value) were taken as a measure for UV-response of the skin (Table 2). In the high flavanol group the Δ-a values determined 24 h after irradiation are approximately 50% and 70% lower after 6 and 12 wk, respectively, of treatment than at the beginning of the study, statistically significant according to the Wilcoxon signed rank test. No significant changes in the 24h a-values and Δ-a values were observed in the low flavanol group during 12 wk treatment. Thus, consumption of a cocoa powder rich in flavanols provides photoprotection, but cocoa powder low in flavanols does not.

**Table 2: Chromametric a-values of skin at day 0, and at wk 6 and wk 12 of the study; decreasing a-values 24 h after irradiation and Δa-values indicate a photoprotective effect.**

| | Time (wk) | | |
|---|---|---|---|
| a-value | 0 | 6 | 12 |
| | | | |

| | *high flavanol group (n=12)* | | |
|---|---|---|---|
| before irradiation | 7.7 ± 2.2 | 8.2 ± 2.0 | 7.9 ± 1.9 |
| 24h after irradiation | 12.5 ± 1.8 | 10.5 ± 2.1* | 9.4 ± 1.8* |
| Δ-a value | 4.8 | 2.3* | 1.5* |
| | | | |

| | *low flavanol group(n=12)* | | |
|---|---|---|---|
| before irradiation | 7.4 ± 1.9 | 8.0 ± 1.6 | 7.4 ± 2.2 |
| 24h after irradiation | 11.1 ± 2.7 | 11.2 ± 2.8 | 11.9 ± 2.8 |
| Δ-a value | 3.7 | 3.2 | 4.5 |

| | | | |
|---|---|---|---|
| *significantly different from wk 0; p < 0.05 | | | |

Cutaneous blood flow. Following supplementation with the HF cocoa powder, an increase in blood flow was observed in cutaneous (1 mm) and subcutaneous (7- 8 mm) tissues (Table 3). In comparison to the starting value, peripheral blood flow was significantly increased about 2-fold (1 mm) and 1.4-fold (7-8 mm) after 12 wk of treatment. No change in blood flow was found in the LF group. The difference between groups was statistically significant comparing the 6 wk and 12 wk values in favor of the HF group. Since blood flow velocity was not affected in both groups it is suggested that the effect is due to a vasodilation of peripheral vessels. There were no significant changes in hemoglobin content.

**Table 3: Peripheral blood flow in skin (1 and 7-8 mm layer).**

| | Time (wk) | | |
|---|---|---|---|
| | 0 | 6 | 12 |
| | | | |
| | *high flavanol group (n=12)* | | |
| Relative blood flow (au) 1mm | 16 ± 7 | 24 ± 12* | 32 ± 16* |
| Blood flow velocity (au) 1mm | 21 ± 13 | 20 ± 13 | 22±9 |
| Relative blood flow (au) 7-8 mm | 133 ± 57 | 155 ± 61* | 183 ± 66* |
| Blood flow velocity (au) 7-8 mm | 35 ± 17 | 34 ± 16 | 43 ± 17 |
| | | | |

| | *low flavanol group(n=12)* | | |
|---|---|---|---|
| Relative blood flow (au) 1mm | 17 ± 9 | 17 ± 6 | 16 ± 6 |
| Blood flow velocity (au) 1mm | 17 ± 7 | 15 ± 4 | 14 ± 5 |
| Relative blood flow (au) 7-8 mm | 144 ± 45 | 134 ± 50 | 131 ± 47 |
| Blood flow velocity (au) 7-8 mm | 30 ± 9 | 27 ± 11 | 27 ± 9 |

| | | | |
|---|---|---|---|
| *significantly different from wk 0; p < 0.05; au = arbitrary units | | | |

Skin structure and texture. Upon supplementation with HF cocoa powder, a moderate but statistically significant increase in density and thickness of the skin was observed (Table 4). For both parameters, no change was found in the LF group. Using the SELS method, a statistically significant decrease in skin roughness was measured in the HF group, whereas no change was found in the LF group. However, scaling was decreased in both groups during intervention. No changes in the SELS parameters smoothness and wrinkles was observed in any intervention group. Skin hydration was significantly increased during the supplementation with HF cocoa, whereas it was not affected in the LF group (Table 4). Transepidermal water loss was significantly decreased in the HF group comparing the starting values with those measured in wk 6 and 12; no difference was found in the LF cocoa powder treatment group.

**Table 4: Skin structure and hydration parameters.**

| | Time (wk) | | |
|---|---|---|---|
| | 0 | 6 | 12 |
| | | | |

| | *high flavanol group (n=12)* | | |
|---|---|---|---|
| Density (pixel) | 10.2 ± 1.7 | 11.3 ± 2.1* | 11.9 ± 1.6* |
| Thickness (mm) | 1.11 ± 0.11 | 1.20 ± 0.14* | 1.24 ± 0.13* |
| Roughness (au) | 0.27 ± 0.20 | 0.20 ± 0.17 | 0.19 ± 0.18* |
| Scaling (au) | 0.14 ± 0.09 | 0.10 ± 0.07 | 0.08 ± 0.06* |
| Smoothness (au) | 20.3 ± 1.9 | 20.9 ± 1.9 | 21.2 ± 2.5 |
| Wrinkles (au) | 42.2 ± 5.1 | 41.8 ± 4.1 | 41.8 ± 4.1 |
| Hydration (au) | 39 ± 4 | 40 ± 6 | 44 ± 8 * |
| Transepidermal water loss (gh⁻¹ m⁻²) | 8.7 ± 3.7 | 7.8 ± 3.5 | 6.3 ± 2.2* |

| | *low flavanol group(n=12)* | | |
|---|---|---|---|
| Density (pixel) | 12.5 ± 1.2 | 12.3 ± 1.4 | 12.4 ± 1.2 |
| Thickness (mm) | 1.05 ± 0.10 | 1.05 ± 0.10 | 1.04 ± 0.11 |
| Roughness (au) | 0.13 ± 0.20 | 0.17 ± 0.17 | 0.15 ± 0.13 |
| Scaling (au) | 0.18 ± 0.22 | 0.11 ± 0.08 | 0.13 ± 0.11 |
| Smoothness (au) | 19.6 ± 3.1 | 20.7 ± 2.1 | 20.5 ± 1.9 |
| Wrinkles (au) | 44.4 ± 5.4 | 44.0 ± 5.1 | 43.7 ± 4.4 |
| Hydration (au) | 38 ± 5 | 36 ± 4 | 36 ± 6 |
| Transepidermal water loss (gh⁻¹ m⁻²) | 7.2 ± 4.2 | 7.4 ± 3.2 | 6.9 ± 2.0 |

| | | | |
|---|---|---|---|
| *significantly different from wk 0; p < 0.05; au = arbitrary units | | | |

## Claims

1. A non-therapeutic method of improving skin quality in a subject by reducing skin roughness, increasing skin thickness or increasing skin density, the method comprising orally administering to the subject a composition which comprises an effective amount of a cocoa component wherein the cocoa component comprises:
(i) a compound selected from epicatechin and catechin; or
(ii) a compound having the formula A, or a pharmaceutically acceptable salt thereof: 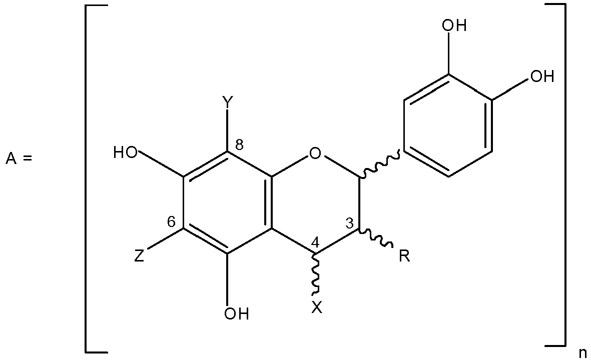 wherein
n is an integer from 2 to 18;
R and X each have either α or ß stereochemistry;
R is OH or O-sugar;
the substituents of C-4, C-6 and C-8 are X, Z and Y, respectively, and bonding of monomeric units occurs at C-4, C-6 or C-8;
when any C-4, C-6 or C-8 is not bonded to another monomeric unit, X, Y and Z independently are hydrogen or a sugar; and
the sugar is optionally substituted with a phenolic moiety at any position, for instance, via an ester bond.

2. A non-therapeutic method according to claim 1, wherein the subject is a veterinary animal.

3. A non-therapeutic method according to claim 1, wherein the subject is a human.

4. A non-therapeutic method according to claim 1, wherein the composition is a food.

5. A non-therapeutic method according to claim 4, wherein the food is a pet food.

6. A non-therapeutic method according to claim 4, wherein the food is a beverage.

7. A non-therapeutic method according to claim 1, wherein the composition is a dietary supplement.

8. A non-therapeutic method according to claim 1, wherein the cocoa component is a cocoa powder, a cocoa extract or chocolate liquor.

9. A non-therapeutic method according to claim 1, wherein the compound is (-)-epicatechin.

10. A non-therapeutic method according to claim 1 wherein the compound is a procyanidin dimer.

## Patentansprüche

1. Nicht-therapeutisches Verfahren zur Verbesserung der Hautqualität in einem Testindividuum durch Verminderung der Haut-Rauheit, Verstärkung der Hautdicke oder Verstärkung der Haut-Dichte, wobei das Verfahren die orale Verabreichung an das Testindividuum einer Zusammensetzung umfasst, die eine wirksame Menge einer Kakao-Komponente umfasst, wobei die Kakao-Komponente folgendes umfasst:
(i) eine Verbindung, die aus Epikatechin und Katechin ausgewählt ist; oder
(ii) eine Verbindung mit der Formel A, oder ein pharmazeutisch verträgliches Salz davon: 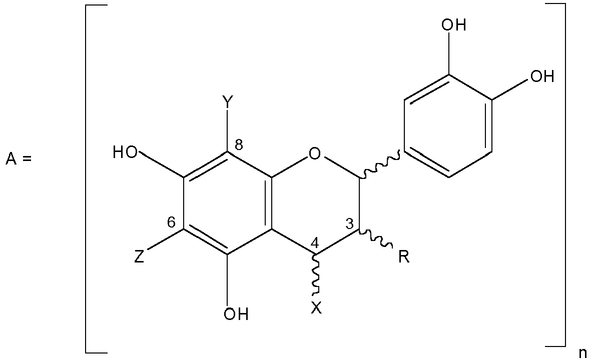 wobei:
n eine ganze Zahl von 2 bi 18 ist;
R und X jeweils entweder eine α- oder β-Stereochemie aufweisen;
R OH- oder O-Zucker ist;
die Substituenten von C-4, C-6 und C-8 X, Z bzw. Y sind, und das Binden von monomeren Einheiten an C-4, C-6 oder C-8 erfolgt;
wenn eines von C-4, C-6 oder C-8 nicht an eine andere monomere Einheit gebunden ist, X, Y und Z unabhängig Wasserstoff oder ein Zucker sind; und
der Zucker gegebenenfalls mit einer Phenol-Einheit an einer beliebigen Position, beispielsweise über eine Esterbindung, substituiert ist.

2. Nichttherapeutisches Verfahren nach Anspruch 1, wobei das Testindividuum ein veterinärmedizinisches Tier ist.

3. Nichttherapeutisches Verfahren nach Anspruch 1, wobei das Testindividuum ein Mensch ist.

4. Nichttherapeutisches Verfahren nach Anspruch 1, wobei die Zusammensetzung ein Nahrungsmittel ist.

5. Nichttherapeutisches Verfahren nach Anspruch 4, wobei das Nahrungsmittel eine Haustiernahrung ist.

6. Nichttherapeutisches Verfahren nach Anspruch 4, wobei das Nahrungsmittel eine Getränk ist.

7. Nichttherapeutisches Verfahren nach Anspruch 1, wobei die Zusammensetzung eine Nahrungsergänzung ist.

8. Nichttherapeutisches Verfahren nach Anspruch 1, wobei die Kakao-Komponente ein Kakaopulver, ein Kakaoextrakt oder Flüssigschokoladen ist.

9. Nichttherapeutisches Verfahren nach Anspruch 1, wobei die Verbindung (-)-Epikatechin ist.

10. Nichttherapeutisches Verfahren nach Anspruch 1, wobei die Verbindung ein Procyanidin-Dimer ist.

## Revendications

1. Procédé non-thérapeutique d'amélioration de la qualité de la peau chez un sujet, par réduction de la rudesse de la peau, augmentation de l'épaisseur de la peau ou augmentation de la densité de la peau, lequel procédé comporte le fait d'administrer au sujet, par voie orale, une composition qui contient, en quantité efficace, un composant de cacao, lequel composant de cacao contient :
i) un composé choisi parmi l'épicatéchine et la catéchine,
ii) ou un composé de formule A, ou un sel pharmacologiquement admissible d'un tel composé : 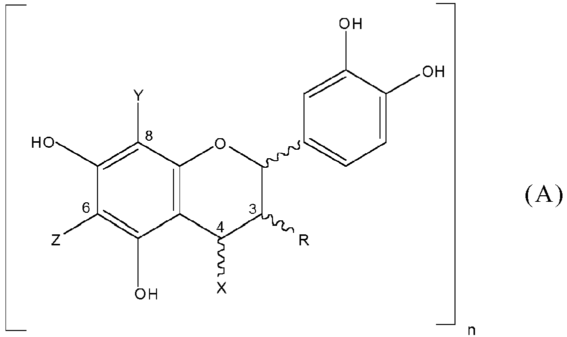 dans laquelle
- l'indice n est un nombre entier valant de 2 à 18 ;
- les entités symbolisées par R et X se trouvent chacune, du point de vue de la stéréochimie, soit en position α, soit en position β ;
- R représente un groupe hydroxyle ou un fragment O-sucre ;
- les substituants placés sur les atomes de carbone C-4, C-6 et C-8 sont respectivement symbolisés par X, Z et Y, et les liaisons entre motifs monomères interviennent au niveau des atomes C-4, C-6 ou C-8 ;
- quand l'un des atomes C-4, C-6 et C-8 n'est pas lié à un autre motif monomère, X, Y et Z représentent, indépendamment, un atome d'hydrogène ou un sucre ;
- et en option, le sucre porte en tant que substituant, en n'importe quelle position, un fragment phénolique, par exemple par l'intermédiaire d'une liaison de type ester.

2. Procédé non-thérapeutique conforme à la revendication 1, dans lequel le sujet est un animal objet de médecine vétérinaire.

3. Procédé non-thérapeutique conforme à la revendication 1, dans lequel le sujet est un humain.

4. Procédé non-thérapeutique conforme à la revendication 1, dans lequel la composition est un aliment.

5. Procédé non-thérapeutique conforme à la revendication 4, dans lequel l'aliment est un aliment pour animaux familiers.

6. Procédé non-thérapeutique conforme à la revendication 4, dans lequel l'aliment est une boisson.

7. Procédé non-thérapeutique conforme à la revendication 1, dans lequel la composition est un supplément diététique.

8. Procédé non-thérapeutique conforme à la revendication 1, dans lequel le composant de cacao est une poudre de cacao, un extrait de cacao ou une pâte de cacao.

9. Procédé non-thérapeutique conforme à la revendication 1, dans lequel le composé est de la (-)-épicatéchine.

10. Procédé non-thérapeutique conforme à la revendication 1, dans lequel le composé est un dimère de procyanidine.
